# EUROPEAN PATENT SPECIFICATION

(11) **EP 1 438 063 B1**
(45) Date of publication and mention of the grant of the patent: **19.05.2010**
(21) Application number: 02799377.3
(22) Date of filing: 26.09.2002
(51) Int. Cl.: A61K 38/06, A61K 31/198, A61P 25/18, A61P 25/28

(54) **GLUTATION PRECURSORS FOR THE TREATMENT OF NEUROPSYCHIATRIC DISORDERS**
GLUTATION VORLAEUFER ZUR BEHANDLUNG VON NEUROPSYCHIATRISSCHEN KRANKHEITEN
DES PRECURSEURS DE LA GLUTATHIONE POUR LE TRAITEMENT DE MALADIES NEUROPSYCHIATRIQUES

(30) Priority: 27.09.2001 US 325061 P
(43) Date of publication of application: 21.07.2004
(73) Proprietor: The Mental Health Research Institute of Victoria, Parkville, Victoria 3052 (AU)
(72) Inventor: BUSH, Ashley, I., Somerville, MA 02143 (US); COPOLOV, David, L., East Malvern, Victoria 3144 (AU); BERK, Michael, Highton, Victoria 3216 (AU)
(74) Representative: Gardner, Rebecca Katherine
(86) International application number: PCT/AU2002/001320
(87) International publication number: WO 2003/026684

(56) References cited:
- WO-A-98/47519
- WO-A1-02/04025
- WO-A1-98/29375
- WO-A2-02/02190
- GB-A- 2 368 339
- US-A- 2002 002 136
- US-B1- 6 329 430
- PARCELL S: "SULFUR IN HUMAN NUTRITION AND APPLICATIONS IN MEDICINE" ALTERNATIVE MEDICINE REVIEW, THORNE RESEARCH INC., SANDPOINT,, US, vol. 7, no. 1, February 2002 (2002-02), pages 22-24, XP008004419 ISSN: 1089-5159
- MAHADIK S P ET AL: "Oxidative stress and role of antioxidant and omega-3 essential fatty acid supplementation in schizophrenia" PROGRESS IN NEURO-PSYCHOPHARMACOLOGY & BIOLOGICAL PSYCHIATRY, OXFORD, GB, vol. 25, no. 3, April 2001 (2001-04), pages 463-493, XP002204382 ISSN: 0278-5846
- KULOGLU M ET AL: "lipid peroxidation and antioxidant enzyme levels in patients with schizophrenia and bipolar disorders" CELL BIOCHEMISTRY AND FUNCTION, BUTTERWORTH, GUILDFORD, GB, vol. 20, 2002, pages 171-175, XP002322140 ISSN: 0263-6484
- RAVAGLIA ET AL: "Antioxidant vitamins and dementia" ARCHIVES OF GERONTOLOGY AND GERIATRICS, ELSEVIER, AMSTERDAM, NL, vol. 26, 1998, pages 431-434, XP005058484 ISSN: 0167-4943

## Description

### FIELD OF THE INVENTION

The present invention relates generally to the modulation of glutathione metabolism in the central nervous system of mammals and to agents for use therein. More particularly, the present invention relates to use of a glutathione precursor in the manufacture of a medicament for the treatment of a neuropsychiatric disorder in a mammal, wherein said glutathione precursor induces, upregulates or otherwise augments antioxidant functional activity in said central nervous system. Up-regulating glutathione metabolism in the central nervous system by up-regulating levels of glutathione precursor molecules is disclosed, which is particularly useful, *inter alia,* in the treatment and/or prophylaxis of conditions characterised by aberrant, unwanted or otherwise inappropriate central nervous system oxidation homeostasis including, but not limited to, schizophrenia.

### BACKGROUND OF THE INVENTION

Bibliographic details of the publications referred to alphabetically by author in the specification are collected at the end of the description.

The reference to any prior art in this specification is not, and should not be taken as, an acknowledgment or any form of suggestion that that prior art forms part of the common general knowledge in Australia.

Schizophrenia is a severe mental illness which affects approximately one person in a hundred. ° Symptoms characterising schizophrenia include delusions (false beliefs of persecution, guilt, grandeur or being under outside control), hallucinations (visual or auditory) and thought disorder (speech which is difficult to follow or jumping from one subject to another with no logical connection). Secondary symptoms of schizophrenia include loss of drive, blunted emotions, social withdrawal and/or lack of insight.

The onset of schizophrenia usually occurs during adolescence or early adulthood, although it has been known to develop in older people. Onset may be rapid, with acute symptoms developing over several weeks, or it may be slow, developing over months or even years.

The causes of schizophrenia are not fully understood. However, during the last few years there has emerged a body of literature which supports an abnormality in oxidation homeostasis systemically and centrally in schizophrenia. The origin of this oxidative stress is still unknown. The brain in schizophrenia exhibits many chemical hallmarks of oxidative attack, in addition to indications of altered antioxidant defence. Any tissue under sustained radical attack may suffer a depletion of the key free radical/H₂O₂ scavenger in the brain, glutathione. Recently, reports have emerged that glutathione is indeed depleted in schizophrenia, and that the antioxidant enzymic activities related to glutathione metabolism are markedly perturbed. Do KQ, Trabesinger AH, Kirsten-Kruger M, Lauer CJ, Dydak U, Hell D, Holsboer F, Boesiger P and Cuenod M., (2000), Euro J Neurosci, 12:3721-8 have reported a significant decrease (-27%) in the cerebrospinal fluid levels of glutathione in drug-free schizophrenia patients compared to controls. This decrease is consistent with the previously reported decrease in the levels of the glutathione metabolyte gamma-glutamylglutamine in the cerebrospinal fluid of such patients (Do KQ, Lauer CJ, Schreiber W, Zollinger M, Gutteck-Amsler U, Cuenod M and Holsboer F., (1995), J Neurochem, 65:2652-62). Furthermore, Do *et al*., (2000) also found a 52% decrease in glutathione levels in the medial prefrontal cortex of schizophrenia patients compared to controls, using a non-invasive proton magnetic resonance spectroscopy method.

Intriguingly, other aspects of the glutathione metabolic pathway are also perturbed in schizophrenia. Decreased peripheral glutathione peroxidase (GPx) activity has been described in schizophrenia patients (Abdalla DS, Monteiro HP, Oliveira JA and Bechara EJ., (1986), Clin Chem, 32:805-7), and the decrease correlates with increased brain atrophy (Buckman TD, Kling AS, Eiduson S, Sutphin MS and Steinberg A., (1987), Biol Psychiatry, 22:1349-56). Plasma GPx positively correlates with psychosis rating scored in schizophrenia patients on or off medication (Yao JK, Reddy RD and van Kammen DP., (1999), Biol Psychiatry, 45:1512-5). GPx is the enzyme that catalyses the scavenging of H₂O₂ and other radicals by glutathione.

These biochemical changes have lead to a call for the critical study of antioxidants as schizophrenia treatments utilised adjunctively with antipsychotic medication. To date, research has focused on the use of indirect means of overcoming the defects in glutathione metabolism such as increasing the efficiency of other radical scavenging systems. For example, Vitamin C, Vitamin E (alpha-tocopherol), alpha-lipoic acid supplements and also selenomethionione have been investigated. Currently, investigators are focusing on the use of Vitamins E and C (Yao *et al.,* 1999, *supra*). Selenomethionione supplementation is well known to augment the activity of glutathione peroxidase (Duffield AJ, Thomson CD, Hill KE and Williams S., (1999), Am J Clin Nutr, 70:896-903). Vitamin E and selenium combined supplementation has already been reported to provide beneficial effects in the treatment of the FALS transgenic mouse model (Gurney ME, Cutting FB, Zhai P, Doble A, Taylor CP, Andrus PK and Hall ED., (1996), Ann Neurol, 39:147-57), demonstrating that the potential antioxidant benefits of such oral supplementation can also be transduced across the blood brain barrier in brain oxidation disorders. However, while being supportive of glutathione metabolism, in that these molecules can function as antioxidants, they are not the most efficient means of increasing glutathione levels in the brain. Accordingly, there is an on-going need to develop methods of treating schizophrenia, either in the form of adjunctive therapies to currently utilised treatments or as a replacement to the use of currently available antipsychotic medication.

In work leading up to the present invention, the inventors have determined that up-regulating glutathione metabolism in the central nervous system, and in particular in the brain, leads to a degree of normalisation of oxidative homeostasis in individuals suffering from schizophrenia and consequently a reduction in the occurrence and/or severity of schizophrenia related symptoms. More particularly, the inventors have determined that the central nervous system oxidative homeostasis, and particularly that of the brain, can be directly normalised via up-regulation of central nervous system glutathione metabolism. The inventors have still further determined that this can be easily and efficiently achieved by administering effective amounts of a glutathione precursor such as N-acetyl cysteine, which molecule is thought to be deacylated in the liver thereby elevating blood cysteine levels, which cysteine can then be transduced across the blood-brain barrier.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

One aspect of the present invention is directed to the use of a glutathione precursor in the manufacture of a medicament for the treatment of a neuropsychiatric disorder in a mammal, wherein said glutathione precursor induces, upregulates or otherwise augments antioxidant functional activity in said central nervous system. The present invention also provides a glutathione precursor for use in the treatment of a neuropsychiatric disorder in a mammal, wherein said glutathione precursor induces, upregulates or otherwise augments antioxidant functional activity in said central nervous system.

Preferably, said neuropsychiatric disorder is schizophrenia, psychosis, bipolar disorder, manic depression, affective disorder, or schizophreniform or schizoaffective disorders, psyschotic depression, drug induced psychosis, delirium, alcohol withdrawal syndrome or dementia induced psychosis. Also preferably, said mammal is a human and said glutathione precursor is N-acetyl cysteine.

Disclosed herein is the up-regulation of glutathione metabolism in the central nervous system of a mammal, comprising administering to said mammal an effective amount of a glutathione precursor.

Disclosed herein is the up-regulation of glutathione metabolism in the brain of a mammal, comprising administering to said mammal an effective amount of a glutathione precursor.

Disclosed herein is the up-regulation of glutathione metabolism in the brain of a mammal, comprising administering to said mammal an effective amount of a glutathione precursor wherein said glutathione metabolism induces, up-regulates or otherwise augments antioxidant functional activity in said brain.

Disclosed herein is the up-regulation of glutathione metabolism in the central nervous system of a mammal, comprising administering to said mammal an effective amount of N-acetyl cysteine

Disclosed herein is the up-regulation of glutathione metabolism in the brain of a mammal, comprising administering to said mammal an effective amount of N-acetyl cysteine

Disclosed herein is the up-regulation of glutathione metabolism in the brain of a mammal, said method comprising administering to said mammal an effective amount of N-acetyl cysteine wherein said glutathione metabolism induces, up-regulates or otherwise augments antioxidant functional activity in said brain.

Disclosed herein is the upregulation of glutathione metabolism in the brain of a human, comprising administering to said human an effective amount of N-acetyl cysteine wherein said glutathione metabolism induces, up-regulates or otherwise augments antioxidant functional activity in said brain.

Disclosed herein is the normalization of oxidative homeostasis in the central nervous system of a mammal, which mammal exhibits unwanted central nervous system oxidation,
comprising administering to said mammal an effective amount of a glutathione precursor
for a time and under conditions sufficient to up-regulate glutathione metabolism.

Disclosed herein is the normalization of oxidative homeostasis in the brain of a mammal, which mammal exhibits unwanted central nervous system oxidation, comprising administering to said mammal an effective amount of a glutathione precursor for a time and under conditions sufficient to up-regulate glutathione metabolism.

Said glutathione precursor may be N-acetyl cysteine and said mammal may be a human.

Disclosed herein is a glutathione precursor for use in the treatment and/or prophylaxis of a condition characterised by:
(i) aberrant, unwanted or otherwise inappropriate oxidative stress; and/or
(ii) inadequate glutathione metabolism,
in the central nervous system of a mammal, comprising administering to said mammal an effective amount of a glutathione precursor.

Disclosed herein is a glutathione precursor for use in the treatment and/or prophylaxis of a condition characterised by:
(i) aberrant, unwanted or otherwise inappropriate oxidative stress; and/or
(ii) inadequate glutathione metabolism,
in the brain of a mammal, comprising administering to said mammal an effective amount of a glutathione precursor

Disclosed herein is a glutathione precursor for use in treating a neuropsychiatric condition in a mammal, comprising administering to said mammal an effective amount of a glutathione precursor for a time and under conditions sufficient to induce, up-regulate or otherwise augment antioxidant functional activity in the brain of said mammal.

Said glutathione precursor may be N-acetyl cysteine.

Said condition may be schizophrenia, psychosis, bipolar disorder, manic depression, affective disorder, or schizophreniform or schizoaffective disorders.

Disclosed herein is a glutathione precursor for use in the treatment and/or prophylaxis of schizophrenia in a mammal, comprising administering to said mammal an effective amount of N-acetyl cysteine for a time and under conditions sufficient to induce, up-regulate or otherwise augment antioxidant functional activity in the brain of said subject.

Disclosed herein is the use of a glutathione precursor in the manufacture of a medicament for the up-regulation of glutathione metabolism and/or normalisation of oxidative homeostasis in the central nervous system of a mammal wherein said glutathione precursor induces, up-regulates or otherwise augments antioxidant functional activity in said central nervous system.

Disclosed herein is the use of a glutathione precursor in the manufacture of a medicament for the treatment of a condition characterised by:
(i) aberrant, unwanted or otherwise inappropriate oxidative stress; and/or
(ii) inadequate glutathione metabolism,
in the central nervous system of a mammal, wherein said glutathione precursor induces, up-regulates or otherwise augments antioxidant functional activity in said central nervous system.

Said central nervous system may be the brain and still more preferably said glutathione precursor may be N-acetyl cysteine.

Said condition may be a neuropsychiatric disorder such as schizophrenia, psychosis, bipolar disorder, manic depression, affective disorder, or schizophreniform or schizoaffective disorders.

Disclosed herein is a pharmaceutical composition comprising a glutathione precursor as hereinbefore defined and one or more pharmaceutically acceptable carriers and/or diluents. Said pharmaceutical composition may additionally comprise molecules with which it is to be co-administered.

Also disclosed are modulatory agents, as hereinbefore defined, when used as disclosed herein.

### BRIEF DESCRIPTION OF THE DRAWINGS

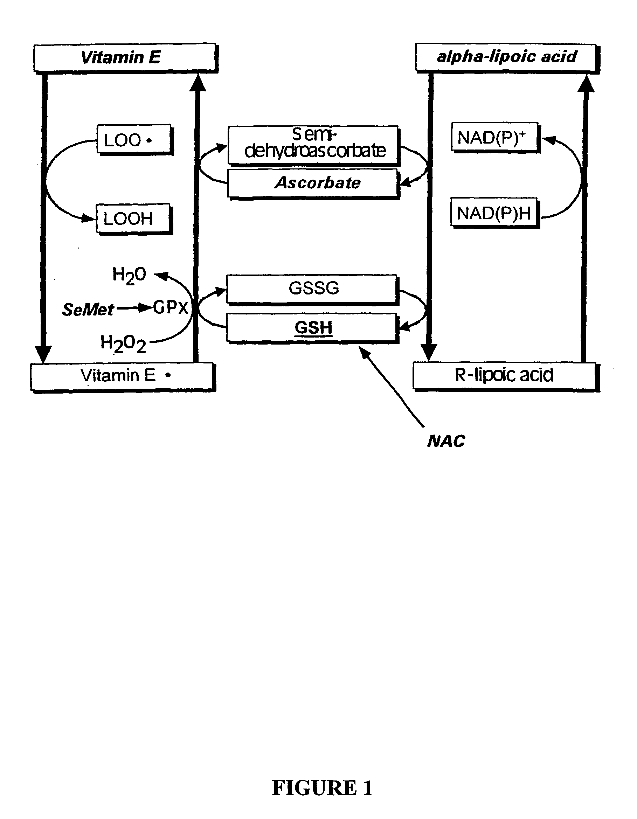

**Figure 1** is a schematic representation of safe oral supplementation which promotes the glutathione radical scavenging biochemical pathway in the treatment of schizophrenia. H₂O₂ generated by abnormal dopamine metabolism is scavenged first by reduced glutathione generating oxidized glutathione (GSSG) in a reaction catalyzed by glutathione peroxidase (GPx). H₂O₂ also contributes to the generation of lipid peroxide (LOO•) radicals in adjacent neurons/synapses. The LOO• radical can propagate through polyunsaturated fatty acids unless scavenged by *vitamin E,* which creates a vitamin E• radical. Vatamin E is then restored by its radical being also reduced by glutathione, again generating oxidized glutathione, and also by *ascorbate,* generating semi-dehydroascorbate. Both of these reducing agents are themselves reduced from their oxidized state by *alpha-lipoic acid* (ALA) (Hagen TM, Ingersoll RT, Lykkesfeldt J, Liu J, Wehr CM, Vinarsky V, Bartholomew JC and Ames AB., (1999), Faseb J, 13:411-8). ALA requires cellular energy to be reduced once it has been oxidized. GPx may not catalyse the reduction of vitamin E radicals by glutathione, but does catalyse the oxidation of glutathione by H₂O₂. *NAC* is the rate limiting precursor for glutathione production. GPx activity is deficient in schizophrenia (Mahadik SP, Mukherjee S, Scheffer R, Correnti EE and Mahadik JS., (1998), Biol Psychiatry, 43:674-9), and oral *selenomethionine* (SeMet) supplementation is a facile means of increasing GPx activity systemically and in the brain (Duffield *et al*., 1999, *supra*).
**Figure 2** is a graphical representation of average body weights (top panel) and daily fluid consumption (bottom panel) of wistar rats receiving normal water or 0.5% or 2% NAC.
**Figure 3** is a graphical representation of baseline locomotor activity of rats receiving either normal water of 0.5% or 2% NAC solutions as drinking water. Data are pre-injection distance moved, averaged over three locomotor experiments ± SEM.
**Figure 4** is a graphical representation of the effect of injection of 0.5 mg/kg of amphetamine (top panel) or 5 mg/kg of amphetamine (bottom panel) on locomotor activity of rats receiving normal water of 0.5% or 2% NAC to drink. Data is expressed as mean change of distance moved per 30 min period ± SEM.
**Figure 5** is a graphical representation of the effect of 0.5 mg/kg of amphetamine on locomotor activity, 30-60 min after injection, of rats receiving either normal water, or 0.5% or 2% NAC to drink. Data are mean change of distance moved ± SEM compared to baseline values before injection.

### DETAILED DESCRIPTION OF THE INVENTION

The present invention is predicated, in part, on the determination both that up-regulation of central nervous system glutathione metabolism, and in particular brain glutathione metabolism, improves aberrant oxidative homeostasis and that it can be achieved via administration of a glutathione precursor, in particular N-acetyl cysteine, to a mammal. Accordingly, this determination now permits the rational design of therapeutic and/or prophylactic methods for treating, either adjunctively or otherwise, conditions characterised by aberrant or otherwise unwanted oxidative homeostasis, or inadequate glutathione metabolism such as schizophrenia.

Accordingly, one aspect of the present invention is directed to the use of a glutathione precursor in the manufacture of a medicament for the treatment of a neuropsychiatric disorder in a mammal, wherein said glutathione precursor induces, upregulates or otherwise augments antioxidant functional activity in said central nervous system. Also disclosed is the up-regulation of glutathione metabolism in the central nervous system of a mammal, comprising administering to said mammal an effective amount of a glutathione precursor

Reference to "central nervous system" should be understood as a reference to that part of the nervous system related to the brain and spinal cord. Preferably, the subject central nervous system region of interest is the brain.

Disclosed herein more particularly is the up-regulation of glutathione metabolism in the brain of a mammal, comprising administering to said mammal an effective amount of a glutathione precursor.

Reference to "glutathione metabolism" should be understood as a reference to any physiological process or pathway which is directly or indirectly regulated by glutathione or metabolic product thereof. "Direct" regulation of a physiological process or pathway by glutathione should be understood as a reference to any process or pathway which is modulated by the functional activity of glutathione or glutathione metabolites such as oxidised glutathione, reduced glutathione or gamma-glutamylglutamine. "Indirect" regulation of a physiological process or pathway should be understood as a reference to any process or pathway which is modulated by the functional activity of a molecule which has itself undergone some form of modulation due to the functional activity of glutathione or a glutathione metabolite as detailed hereinbefore.

Without limiting the present invention to any one theory or mode of action, the physiological importance of glutathione is thought to be dependent on the highly reactive sulphydryl group which is present in the molecule. The easy oxidation of this group to the corresponding disulphide allows participation of glutathione in oxidation-reduction systems. Accordingly, glutathione is thought to play an important role in the protection of cells and tissues from oxidative damage. For example, glutathione is known to function as a scavenger of free radicals and H₂O₂. In this regard, the scavenging functions of glutathione are thought to be catalysed by glutathione peroxidase.

In accordance with the uses disclosed herein, the subject "glutathione metabolism" is preferably that which directly or indirectly results in up-regulation of antioxidant functional activity. However, it should be understood that regulation of glutathione metabolism for purposes other than modulating oxidative homeostasis are nevertheless encompassed by the uses disclosed herein. In terms of the preferred embodiment of the present invention, the subject antioxidant functional activity should be understood as a reference to all forms of antioxidant functional activity which are either directly or indirectly modulated by glutathione or metabolites derived therefrom.

Also disclosed is the upregulation of glutathione metabolism in the brain of a mammal, comprising administering to said mammal an effective amount of a glutathione precursor wherein said glutathione metabolism induces, up-regulates or otherwise augments antioxidant functional activity in said brain.

Without limiting the present invention to any one theory or mode of action, glutathione is a tripeptide containing a sulphydryl group which is widely distributed in living tissue. It is also known by the alternative name of α-glutamylcysteinylglycine or the abbreviation GSH. Glutathione is generally formed as a result of the actions of specific enzymes and not as a direct result of the usual processes of peptide synthesis, being transcription and translation of a nucleic acid molecule specifically encoding said peptide. Glutathione is a molecule of the formula COOHCH(NH₂)CH₂CH₂CONHCH(CH₂SH)CONHCH₂COOH. The first step in the synthesis of glutathione is the formation of a peptide linkage between the gamma-carboxyl group of glutamate and the amino group of cysteine to form ganmma-glutamyl-cysteine. This is catalysed by gamma-glutamylcysteinesynthetase. Formation of this peptide bond requires activation of the gamma-carboxyl group, which activation is provided by ATP. The resulting molecule is an intermediate which is then attacked by the amino group of cysteine. In this second step, which is catalysed by glutathione synthetase, ATP activates the carboxyl group of cysteine to enable it to condense with the amino group of glycine. Accordingly, glutathione is a molecule which is formed subsequently to the actions of enzymes on the rate limiting precursor cysteine. Glutathione cycles between a reduced thiol form (GSH) and an oxidised form (GSSG) in which two tripeptides are linked by a disulfide bond.

In this regard, reference to a "glutathione precursor" should be understood as a reference to any molecule from which glutathione can be directly or indirectly derived. The subject molecule may be naturally or non-naturally occurring. Modification of a molecule in a single step to form glutathione is an example of glutathione being directly derived from a precursor. Modification of a molecule to form an "intermediate" molecule, which intermediate molecule undergoes further modification to form glutathione is an example of glutathione being indirectly derived from the subject precursor. Cysteine is a naturally occurring precursor from which glutathione is indirectly derived. Specifically, cysteine is catalysed to form γ-glutamyl cysteine prior to catalysis of this molecule to take up glycine and thereby form glutamine.

In accordance with a further aspect of the present invention, the inventors have determined that the acylated derivative of cysteine, N-acetyl cysteine, which is orally bioavailable, can supplement glutathione levels in the brain. Accordingly, in a preferred embodiment said glutathione precursor is N-acetyl cysteine.

Disclosed herein is the up-regulation of glutathione metabolism in the central nervous system of a mammal, comprising administering to said mammal an effective amount of N-acetyl cysteine

More particularly, there is disclosed the up-regulation of glutathione metabolism in the brain of a mammal, comprising administering to said mammal an effective amount of N-acetyl cysteine

Still more particularly, there is disclosed the up-regulation of glutathione metabolism in the brain of a mammal, comprising administering to said mammal an effective amount of N-acetyl cysteine
wherein said glutathione metabolism induces, up-regulates or otherwise augments antioxidant functional activity in said brain.

The molecules which may be administered to a mammal in accordance with the present invention may also be linked to a targeting means such as a monoclonal antibody, which provides specific delivery of these molecules to target regions.

The subject of the glutathione precursor administration may be any mammal including, but not limited to, humans, primates, livestock animals (e.g. horses, cattle, sheep, pigs and donkeys), laboratory test animals (e.g. mice, rats, rabbits, guinea pigs), companion animals (e.g. dogs, cats) or captive wild animals (e.g. kangaroos, deer, foxes). Preferably, the mammal is a human.

Disclosed herein is the up-regulation of glutathione metabolism in the brain of a human, comprising administering to said human an effective amount of N-acetyl cysteine wherein said glutathione metabolism induces, up-regulates or otherwise augments antioxidant functional activity in said brain.

Without limiting the present invention to any one theory or mode of action, glutathione is thought to protect against oxidative damage due to its capacity to function both as a scavenger of free radicals and as a substrate for the detoxification of H₂O₂, peroxynitrite, lipid peroxides and a variety of electrophilic compounds including redox-active metal ions (Freedman JH, Ciriolo MR and Peisach J., (1995), J Neurosci, 264:5598-605). Schizophrenia is a disease condition which is characterised by oxidative stress. As detailed hereinbefore, emerging evidence suggests an abnormality in oxidation homeostasis both systemically and centrally in schizophrenia. Specifically, it is thought that abnormalities in an individual's dopamine metabolism leads to the production of free radicals and H₂O₂. These events, coupled with a loss in oxidative defences at sites of dopamine activity in the brain, may lead to the oxidative attack of neuronal structures, thereby resulting in neural dysfunction and giving rise to the psychosis associated with schizophrenia. The inventors have determined that increasing glutathione levels in the brain results in a decrease in oxidative stress due to normalisation of brain oxidative homeostasis and therefore a decrease in the occurrence and/or severity of symptoms associated with schizophrenia. Still further, it has been determined that this normalisation of brain oxidative homeostasis is partly due to the elevation of glutathione preventing the breakdown of dopamine.

Accordingly, disclosed herein is the normalization of oxidative homeostasis in the central nervous system of a mammal, which mammal exhibits unwanted central nervous system oxidation, comprising administering to said mammal an effective amount of a glutathione precursor for a time and under conditions sufficient to up-regulate glutathione metabolism.

More particularly, there is disclosed the normalization of oxidative homeostasis in the brain of a mammal, which mammal exhibits unwanted central nervous system oxidation, comprising administering to said mammal an effective amount of a glutathione precursor for a time and under conditions sufficient to up-regulate glutathione metabolism.

Preferably said glutathione precursor is N-acetyl cysteine and still more preferably said mammal is a human.

Reference to "normalising" oxidative homeostasis should be understood as a reference to improving oxidative homeostasis relative to the oxidative homeostasis status prior to treatment. The subject improvement may be partial or complete. Preferably, said normalisation is a decrease in oxidation in the central nervous system.

The use of N-acetyl cysteine in accordance with the present invention and the uses disclosed herein is particularly desirable since it has been determined that oral administration of N-acetyl cysteine, which is highly convenient, does result in the elevation of glutathione levels in the brain and therefore up-regulation of glutathione metabolism. N-acetyl cysteine rapidly and safely increases plasma cysteine levels due to rapid deacylation of N-acetyl cysteine in the liver to form cysteine. It should be understood, however, that the present invention is not intended to be limited to NAC to cysteine conversion occurring in the liver. Rather, the present invention should be understood to encompass the occurrence of such conversions in any suitable file, such as, for example, the brain.

The development of the use of the present invention has facilitated the design and application of therapeutic and prophylactic protocols for treating conditions characterised by aberrant, unwanted or otherwise undesirable oxidative stress and/or inadequate glutathione metabolism in the central nervous system and, in particular, the brain. Reference to "oxidative stress" should be understood as a reference to oxidation.

Reference to the treatment and/or prophylaxis of the subject condition should be understood as a reference to the treatment of any disease, injury or other condition, the symptoms, cause or side effects of which include aberrant, unwanted or otherwise undesirable oxidative stress and/or inadequate glutathione metabolism in the central nervous system. It should also be understood to encompass reference to conditions in which one or more components or steps which would lead to aberrant, unwanted or otherwise undesirable oxidative stress and/or glutathione metabolism in the central nervous system have occurred but which may not yet have resulted in noticeable symptoms. This includes, for example, conditions which occur as a side effect of a treatment regime for an unrelated disease condition.

Preferably, the subject condition is a neuropsychiatric disorder and still more preferably schizophrenia, psychosis, bipolar disorder, manic depression, affective disorder, or schizophreniform or schizoaffective disorders, psychotic depression, drug induced psychosis, delirium, alcohol withdrawal syndrome or dementia induced psychosis.

The use of the present invention preferably facilitates the subject condition being reduced, retarded or otherwise inhibited. Reference to "reduced, retarded or otherwise inhibited" should be understood as a reference to inducing or facilitating the partial or complete inhibition of any one or more causes or symptoms of the subject condition. In this regard, it should be understood that conditions such as neuropsychiatric disorder are extremely complex comprising numerous physiological events which often occur simultaneously. In terms of the object of the subject use for treatment and/or prophylaxis, it should be understood that the present invention contemplates both relieving any one or more symptoms of the subject condition (for example, relieving one or more psychosis events) or facilitating retardation or cessation of the cause of the disease condition (for example, reducing oxidative stress thereby minimising any further neuronal damage.

Accordingly, disclosed herein is a glutathione precursor for use in the treatment and/or prophylaxis of a condition characterised by:
(i) aberrant, unwanted or otherwise inappropriate oxidative stress; and/or
(ii) inadequate glutathione metabolism,
in the central nervous system of a mammal, comprising administering to said mammal an effective amount of a glutathione precursor

More particularly disclosed herein is a glutathione precursor for use in the treatment and/or prophylaxis of a condition characterised by:
(i) aberrant, unwanted or otherwise inappropriate oxidative stress; and/or
(ii) inadequate glutathione metabolism,
in the brain of a mammal, comprising administering to said mammal an effective amount of a glutathione precursor

Reference to "aberrant, unwanted or otherwise inappropriate" oxidative stress should be understood as a reference to excessive oxidation or to a physiologically normal level of oxidation, which level is inappropriate in the given circumstances or otherwise unwanted. It should also be understood that the subject unwanted oxidative stress may occur as a side-effect or other indirect consequence of a treatment regime for an unrelated condition, for example, due to the effect of anti-tumour drugs, radiotherapy, dopamine replacement therapy for Parkinson's disease or anti-psychotic medication.

Still more particularly, the present invention relates to a glutathione precursor for use in the treatment of a neuropsychiatric disorder in a mammal, wherein said glutathione precursor induces, upregulates or otherwise augments antitoxidant functional activity in said central nervous system.

Preferably said glutathione precursor is N-acetyl cysteine.

More preferably said condition is schizophrenia, psychosis, bipolar disorder, manic depression, affective disorder, or schizophreniform or schizoaffective disorders.

Most preferably said condition is schizophrenia.

Administration of the glutathione precursor (herein referred to as the "modulatory agent"), in the form of a pharmaceutical composition, may be performed by any convenient means. The modulatory agent of the pharmaceutical composition is contemplated to exhibit therapeutic activity when administered in an amount which depends on the particular case. The variation depends, for example, on the human or animal and the form of modulatory agent chosen. A broad range of doses may be applicable. Considering a patient, for example, from about 0.1 mg to about 1 mg of modulatory agent may be administered per kilogram of body weight per day. Dosage regimes may be adjusted to provide the optimum therapeutic response. For example, several divided doses may be administered daily, weekly, monthly or other suitable time intervals or the dose may be proportionally reduced as indicated by the exigencies of the situation. The modulatory agent may be administered in a convenient manner such as by the oral, intravenous (where water soluble), intraperitoneal, intramuscular, subcutaneous, intradermal or suppository routes or implanting (e.g. using slow release molecules). Preferably the agent is administered orally and dosages of 1-10 grams per day are envisioned. More particularly the dosage is 2-6 grams per day. The modulatory agent may be administered in the form of pharmaceutically acceptable nontoxic salts, such as acid addition salts or metal complexes, e.g. with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be administered in tablet form, the tablet may contain a binder such as tragacanth, corn starch or gelatin; a disintegrating agent, such as alginic acid; and a lubricant, such as magnesium stearate.

Routes of administration include, but are not limited to, respiratorally, intratracheally, nasopharyngeally, intravenously, intraperitoneally, subcutaneously, intracranially, intradermally, intramuscularly, intraoccularly, intrathecally, intracereberally, intranasally, infusion, orally, rectally, *via* IV drip patch and implant. Preferably, said route of administration is oral.

In a most preferred embodiment, the present invention provides N-acetyl cysteine for use in the treatment of schizophrenia in a mammal, wherein said N-acetyl cysteine induces, up-regulates or otherwise augments antioxidant functional activity in the central nervous system of said subject.

Also disclosed herein is the use of a glutathione precursor in the manufacture of a medicament for the up-regulation of glutathione metabolism and/or normalisation of oxidative homeostasis in the central nervous system of a mammal wherein said glutathione precursor induces, up-regulates or otherwise augments antioxidant functional activity in said central nervous system.

Preferably said central nervous system is the brain.

Still more preferably, said glutathione precursor is N-acetyl cysteine.

As detailed hereinbefore, the inventors have determined that elevation of glutathione levels in a subject leads to normalisation of oxidative homeostasis, at least in part, by preventing the breakdown of dopamine. Accordingly, in a related aspect, the methods and uses disclosed herein are also useful for treating the symptoms of dopamine depletion, which are often observed in conditions such as schizophrenia, including apathy and inertia. In this regard, it should be understood that the occurrence of such dopamine-depletion related symptoms falls within the scope of a condition characterised by "aberrant, unwanted or otherwise inappropriate oxidative stress" since it is characterised by a cycle of excessive dopamine breakdown.

Also disclosed herein is the use of a glutathione precursor in the manufacture of a medicament for the treatment of a condition characterised by:
(i) aberrant, unwanted or otherwise inappropriate oxidative stress; and/or
(ii) inadequate glutathione metabolism,
in the central nervous system of a mammal, wherein said glutathione precursor induces, up-regulates or otherwise augments antioxidant functional activity in said central nervous system.

Preferably said central nervous system is the brain and still more preferably said glutathione precursor is N-acetyl cysteine.

Most preferably said condition is a neuropsychiatric disorder and still more preferably schizophrenia, psychosis, bipolar disorder, manic depression, affective disorder, or schizophreniform or schizoaffective disorders.

In a related aspect of the present invention the mammal undergoing treatment may be human or an animal in need of therapeutic or prophylactic treatment.

Reference herein to "treatment" and "prophylaxis" is to be considered in its broadest context. The term "treatment" does not necessarily imply that a mammal is treated until total recovery. Similarly, "prophylaxis" does not necessarily mean that the subject will not eventually contract a disease condition. Accordingly, treatment and prophylaxis include amelioration of the symptoms of a particular condition or preventing or otherwise reducing the risk of developing a particular condition. The term "prophylaxis" may be considered as reducing the severity of onset of a particular condition. "Treatment" may also reduce the severity of an existing condition or the frequency of acute attacks (for example, reducing the severity of psychotic episodes).

In accordance with these uses, the modulatory agent defined herein, preferably a glutathione precursor may be coadministered with one or more other compounds or molecules. By "coadministered" is meant simultaneous administration in the same formulation or in two different formulations via the same or different routes or sequential administration by the same or different routes. By "sequential" administration is meant a time difference of from seconds, minutes, hours or days between the administration of the two types of molecules. These molecules may be administered in any order.

In this regard, although N-acetyl cysteine supplementation is thought to be the most direct and efficient means of achieving the object of the present invention, this method may nevertheless be augmented through the use of indirect means of increasing the efficiency of other radical scavenging systems. For example, N-acetyl cysteine therapy maybe combined with the administration of supplements that bolster indirect participants in glutathione metabolism. These indirect participants of glutathione metabolism are herein referred to as "para"-glutathione antioxidant systems since radicals propagate radially in terms of the biochemical chain reaction. Accordingly, glutathione, while central to the antioxidant pool, is not the sole means of removing radicals. Examples of agents which may be co-administered to augment normalisation of oxidative homeostasis include Vitamin C, Vitamin E (alpha-tocopherol), alpha-lipoic acid supplements and selenomethionione.

Without limiting the present invention in any way, selenomethionione supplementation is known to augment the activity of glutathione peroxidase, the enzyme that catalyses the scavenging of radicals and H₂O₂ by glutathione. Alpha-lipopic acid has been shown to facilitate the restoration of glutathione from oxidised glutathione and has been observed to attenuate oxidative damage in rodents. The actions of other supplements are schematically detailed in Figure 1. Vitamin E and selenomethionine supplementation are particularly desired since oral supplementation has been found to result in transduction of these molecules across the blood brain barrier in brain oxidation disorder. Suitable dosage regimes of the supplemental molecules can be determined by the person of ordinary skill in the art.

The uses of the present invention may also be combined with currently known treatment such as the administration of anti-psychotic drugs.

Also disclosed herein is a pharmaceutical composition comprising a glutathione precursor as hereinbefore defined and one or more pharmaceutically acceptable carriers and/or diluents. Said pharmaceutical composition may additionally comprise molecules with which it is to be co-administered. These agents are referred to as the active ingredients.

Although the use of the present invention is preferably achieved via the oral administration of a glutathione precursor, it should be understood that the present invention is not limited to this route of administration and may encompass any other suitable routes of administration. In this regard, the pharmaceutical forms suitable for injectable use include sterile aqueous solutions (where water soluble) or dispersions and sterile powders for the extemporaneous preparation of sterile injectable solutions or dispersion or may be in the form of a cream or other form suitable for topical application. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilisation. Generally, dispersions are prepared by incorporating the various sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1 % by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 0.1 µg and 3000 mg of active compound.

The tablets, troches, pills, capsules and the like may also contain the components as listed hereafter: a binder such as gum, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.
The pharmaceutical composition may also comprise genetic molecules such as a vector capable of transfecting target cells where the vector carries a nucleic acid molecule encoding a modulatory agent. The vector may be, for example, a viral vector.

Also disclosed herein are modulatory agents, as hereinbefore defined, when used in the uses disclosed herein.

The present invention is further described by the following examples.

### EXAMPLE 1

### N-ACETYL CYSTEINE TREATMENT AND THE BEHAVIOURAL EFFECT OF AMPHETAMINE IN RATS

### Methods

### Animals

Male Sprague Dawley rats (380-430g) were obtained from Department of Pathology, University of Melbourne. On arrival rats were housed in pairs in a temperature-controlled (20-24°C) colony room maintained on a 12hr light-dark cycle (6am-6pm). Rats had continuous access to food and water (plain or treated) and were weighed on arrival and then daily from onset of experiment. Rats were allowed to adjust to the new environment for 3 days before starting the experiment.

### Drug treatment

This experiment consisted of 24 rats in total. Eight rats received plain drinking water (controls), 8 rats received 0.5% NAC (Sigma) in their drinking water (low dose) and 8 rats received 2% NAC in their drinking water (high dose). Drug treatment was administered for 14 consecutive days. Bottles were cleaned and fresh drug solution was made up on days 4, 8 and 11. Body weights and amount of drinking water consumed per box were measured on a daily basis. After 7 days of drug treatment, behavioural testing began and continued until day 14 of treatment.

### Locomotor activity

Locomotor activity was assessed in 8 automated photo cell cages, data being collected in 5-min intervals. Animals were placed individually in cages and baseline activity was measured during the first 30 minutes. After 30 minutes rats received a subcutaneous injection of either saline (vehicle), 0.5 mg/kg or 5 mg/kg amphetamine and locomotor activity was measured for a further 90 minutes. Drug treatment and time of day of the experiment were both randomised (see table 1). Each rat received 3 locomotor sessions in total. A period of 3 days was allowed between locomotor experiments to ensure complete elimination of amphetamine.

### Statistics

Locomotor hyperactivity was obtained as total distance moved per 30 min, i.e. before injection and 0-30 min, 30-60 min and 60-90 min after injection. In order to account for slight variations in baseline activity, post-injection behaviour was expressed a change of baseline activity. Average data for rats receiving water, 0.5% NAC, or 2% NAC was compared using analysis of variance for repeated measures (ANOVA) using the Systat 9.0 software package.

### Results

### Body weights and water consumption

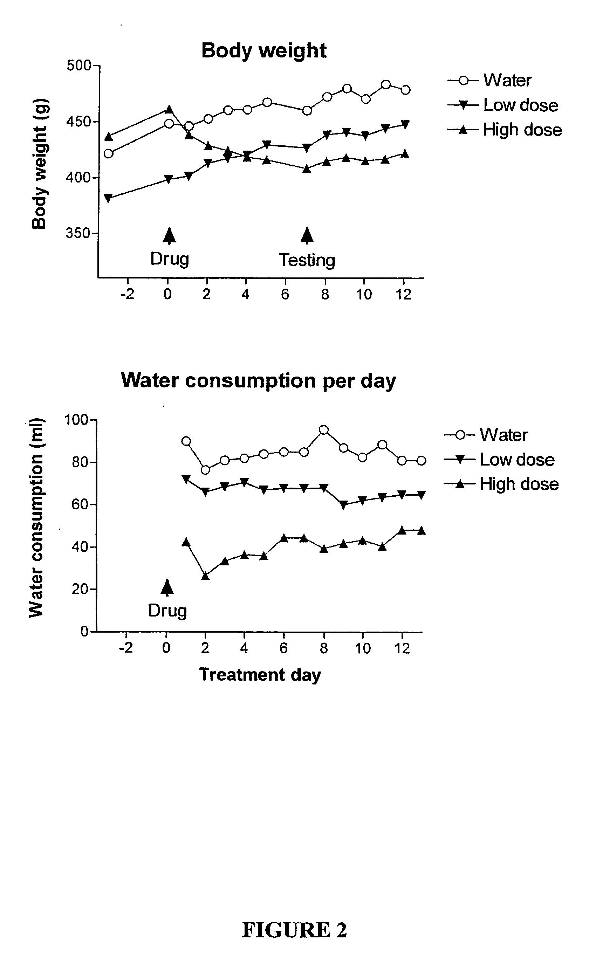

Average daily water consumption per box during the 14 days of treatment was considerably lower when the rats received the 2% dose of NAC, compared to the 0.5% dose and water controls. Water consumption ranged from 77-90 ml in control rats, 60-72 ml in low dose rats and 27-49 ml in high dose rats (figure 2). This was accompanied by a marked decrease in body weight over the treatment period of the high dose rats compared with controls (figure 2, table 2).

### Effect of amphetamine on locomotor activity

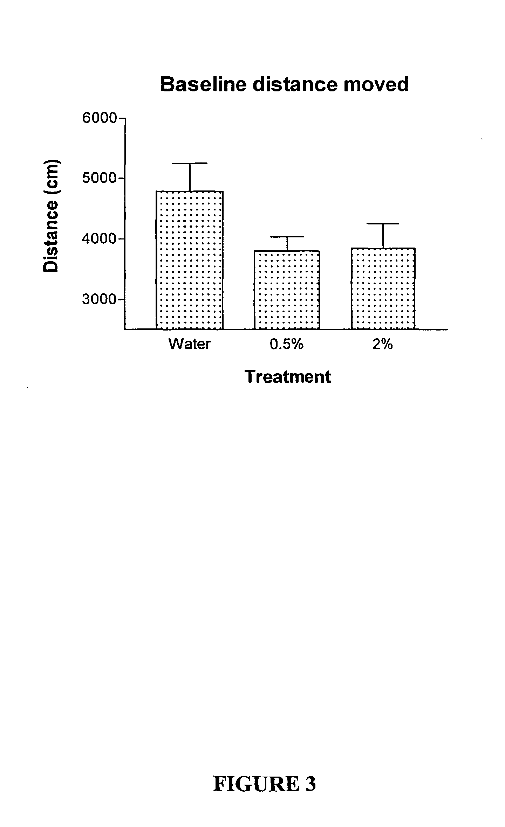

All rats were tested three times: once after saline injection, once after injection of 0.5 mg/kg of amphetamine, and once after injection of 5 mg/kg of amphetamine (table 2). Baseline locomotor activity before injection, averaged over these three sessions, tended to be lower in NAC-treated rats than in water controls, but this difference did not reach statistical significance (figure 3).

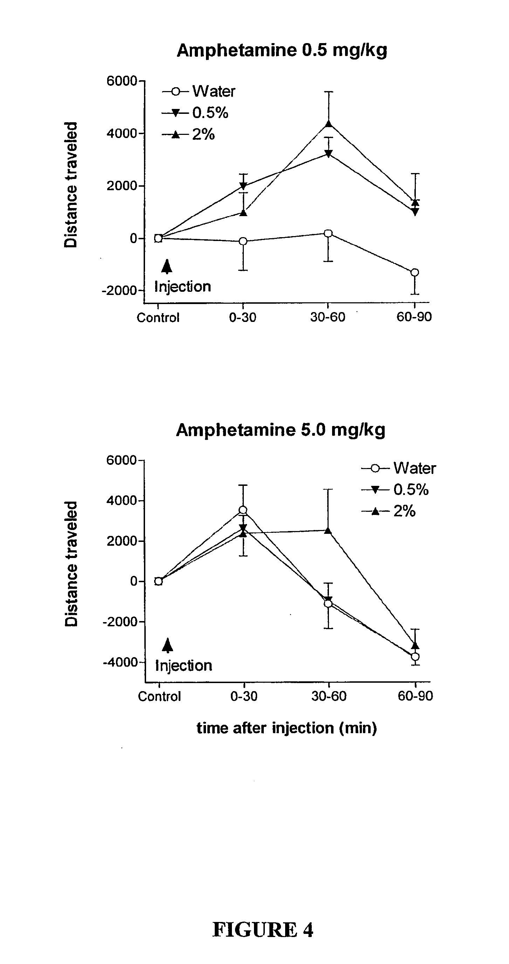

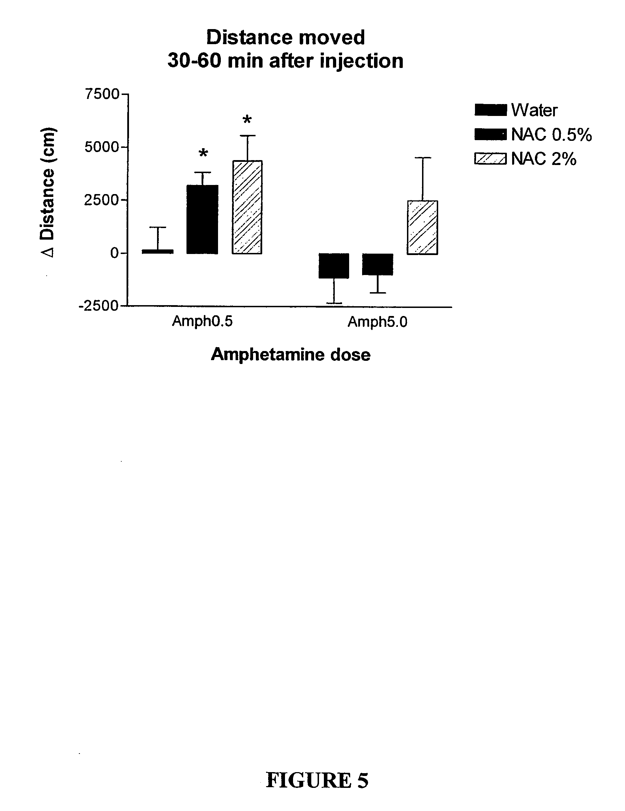

After injection of saline, as expected locomotor activity fell to a low level throughout the remaining 90 min of the experiment. In contrast, injection of 0.5 mg/kg of amphetamine induced a behavioural hyperactivity, with values for distance moved after injection equalling or exceeding the initial spontaneous activity (figure 3). The overall effect of 0.5 mg/kg of amphetamine tended to be greater in rats receiving either 0.5% NAC (F_{(1,14)}=5.1 1, P=0.04) or 2% NAC (F_{(1,13)}=4.3, P=0.06) in the drinking water. The maximal effect of this dose of amphetamine was observed 30-60 min after injection (figure 4). This maximal effect was significantly greater in rats receiving either NAC dose compared to water controls (figure 5).

Injection of 5 mg/kg of amphetamine initially induced marked behavioural hyperactivity, but activity fell to low levels later in the 90 min observation period. The overall effect of 5 mg/kg of amphetamine tended to be greater in rats receiving 2% NAC to drink, but this difference failed to reach overall statistical significance. There was, however an overall significant interaction of treatment x time, reflecting the differing time-course of the effect of this dose of amphetamine between groups. This interaction was significant when comparing rats receiving 2% NAC with controls (F_{(2,28)}=4.9, P=0.024), but not when comparing rats receiving 0.5% NAC with controls. Thus, in rats receiving the 2% NAC dose, locomotor hyperactivity was maintained longer than in controls (figure 4).

### Analysis

The effect of amphetamine given to rats is to increase locomotor activity mediated by increased dopamine release in the nucleus accumbens in the ventral forebrain. The current results show that NAC-treatment enhances the effect of amphetamine. This was seen as a greater maximal effect of 0.5 mg/kg of amphetamine in rats receiving either 0.5% NAC or 2% NAC, and as a prolonged effect of 5 mg/kg of amphetamine in rats receiving 2% NAC. These results confirm that NAC impacts upon the central neurometabolism of DA, making it more available in the nucleus accumbens. NAC, after being metabolised to cysteine in the liver, is then converted to glutathione (GSH), which passes into the brain through the blood brain barrier. GSH scavenges hydrogen peroxide and other pro-oxidants, and prevents oxidation and breakdown of biochemicals like dopamine. By increasing the availability of GSH in the brain, less of the dopamine that is released by the action of amphetamine would be oxidized and broken down. This would make more dopamine available to stimulate postsynaptic receptors, leading to the enhanced or prolonged behavioural effects which were observed.

These findings confirm that oral NAC treatment favourably impacts upon brain dopamine metabolism.

### EXAMPLE 2

### N-ACETYL CYSTEINE IN SCHIZOPHRENIA: A DOUBLE-BLIND, RANDOMISED, PLACEBO-CONTROLLED TRIAL

This study investigates a novel, tolerable and practical adjunctive therapy. In this study the efficacy and tolerability 3g daily of N-acetyl Cysteine (NAC) is compared to placebi in patients who are suffering from both acute and chronic schizophrenia and are on treatment with the atypical antipsychotic drugs olanzapine, risperidone and clozapine.

### (i) Study Group

Two hundred patients aged 18-65 years meeting DSM-IV criteria for schizophrenia on a structured clinical interview (SCID) are studied. There is a group of 100 patients with chronic, stable schizophrenia on clozapine as well as 100 patients suffering from an acute relapse of schizophrenia, who are with risperidone and olanzapine. The patients are assigned randomly and consecutively to treatment with NAC or placebo in a double blind fashion.

### (ii) Inclusion and Exclusion Criteria

To be included the patients are required to meet DSM-IV criteria for schizophrenia. The age is between 18 to 65, and both males and females are studied. The study population includes both inpatients and outpatients. Patients with abnormal renal, hepatic, throid or haematalogical findings are excluded from the study, as are patients who have an acute systemic medical disorder. Subjects who have had a neuroleptic depot preparation in the last month are excluded, and patients on psychoactive medications for other indications need to have been on those agents for at least 1 month. Patients on mood stabilisers (lithium, valproate and carbamazepine) are excluded from the study. Also excluded are those patients who are unable to comply with either the requirements of informed consent or the treatment protocol.

### (iii) Measurements

The patients are assessed on admission through a structural clinical interview (SCID, DSM-IV). A complete physical as well as a neurological examination is also done. The patient's psychiatric condition is measured on admission using the Positive and Negative Symptom Scale (PANSS) Clinical Global Impression (CGI) improvement and severity scales, as well as the Global Assessment of Functioning Scale (GAF). In addition, the AIMS, Simpson-Angus and Barnes Akathisia scales is performed. These scales are repeated two weekly for eight weeks or on the day of study termination if the patient withdraws prior to 8 weeks. A further extension phase under continued double blind treatment follows, with monthly evaluations to a total of 6 months. Adverse events are tabulated. Clozapine, olanzapine and risperidone blood level monitoring is done at baseline and at the end of 2 months of treatment to exclude potential drug interactions. Routine laboratory investigations assess renal, thyroid, haematological and hepatic function. A urine analysis is done. Vital signs are monitored at each visit (blood pressure, pulse, weight).

### (iv) Study Procedure

All patients are treated with either olanzapine or risperidone (acute treatment group) or clozapine (chronic treatment group) prior to randomisation. All randomised patients receive NAC 4 capsules BD to a total dose of 3g daily, or placebo. Platelet glutamate receptor sensitivity, measured using spectrofluorometry, is done at baseline and at the end of the first and second month. All patients give written informed consent before enrolment. Patients are withdrawn from the study if they withdraw consent or develop serious adverse events associated with the study drug. Discontinuation due to adverse events is either at the request of the patient or the discretion of the investigator. The trial is approved by relevant research and ethics committees. The trial is conducted according to GCP guidelines.

### (v) Rationale for Dosage Strategy

NAC is well tolerated by humans and used in the clinic as a mucolytic (for AIDS and cystic fibrosis treatment), for treatment of acetaminophen overdose where it prevents glutathione depletion in the liver, and is also available in the US as an over-the-counter supplement. Human dosing can be up to 5 g/day without adverse effects (Louwerse ES, Weverling GJ, Bossuyt PM, Meyjes FE and de Jong JM., (1995), Arch Neurol, 52:559-64). The capsules of NAC are 750 mg, and the maximum dose in a clinical trial so far has been 5 g/day. A dose of 3 g/day (4 capsules, 2 bid) sustains elevated plasma cystine levels.

### (vi) Analysis of Results

Overall power to detect significant differences between the actual pattern of means; assuming a correlation of post-treatment scores with baseline measurements of 0.7 and an effect of the NAC adjunctive treatment such that the NAC group differs from controls by 0.75 standard deviations, power is maintained above 90% with 50 subjects in each group. Pairwise comparisons with 50 subjects per group enables effects smaller than 0.6 standard deviations to be detected with power of 80%. These effect sizes are in the small to moderate range. The experiment is this capable of detecting difference between groups of clinical and scientific interest.

### (vii) Available Patient Population

The trial is conducted in three sites over three years, Geelong and Werribee in Victoria Australia as well as in Johannesburg, South Africa. All three centres have significant numbers of patients who meet trial criteria. Geelong has 250 patients on clozapine, Werribee 100 and Johannesburg many hundreds. Similarly, acute relapse of schizophrenia is amongst the most common clinical problems seen in all centres.

### EXAMPLE 3

### RATIONALE FOR DOSAGES OF SUPPLEMENTARY AGENTS

### Selenomethionione

Tables are purchased with 200 ug. One per day is an acceptable long-term dose with no known adverse effects.

### Vitamin E

400 IU of alpha-tocopherol is sufficient to increase CSF levels without causing adverse effects. Doses higher than 2000 IU/day may cause coagulopathies and can be a source of radicals itself (Bowry VW, Mohr D, Cleary J and Stocker R., (1995), J Biol Chem, 270:5756-63)

### Vitamin C

500 mg of Vitamin C daily is sufficient to elevate plasma levels 60%. Excess Vitamin C may increase nucleic acid oxidation (Podmore ID, Griffiths HR, Herbert KE, Mistry N, Mistry P and Lunec J., (1998), Nature, 392:559)

### Alpha-lipoic Acid

100 mg tablets are marketed. Doses of 600-1200 mg/day for three weeks have been shown to be tolerated with no adverse effects, and with reproducibly demonstrated benefit in decreasing the symptoms of diabetic neuropathy (Ziegler D and Gries FA., (1997), Diabetes, 46 Suppl 2, S62-) (believed to be caused by oxidative radical damage).

Therefore we will use 600 mg/day in divided doses.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

**Table 2: randomisation table for the locomotor hyperactivity experiments. Rats 1-8 received normal water, rats 9-16 chronically received 0.5% NAC in their drinking water, and rats 17-24 received 2% NAC. During the locomotor hyperactivity experiments, the animals were injected with either saline (SAL), 0.5 mg/kg of amphetamine (0.5), or 5 mg/kg of amphetamine (5).**

| **Rat#** | Injection | | |
|---|---|---|---|
| | **1^{st} dose** | **2^{nd} dose** | **3^{nd} dose** |
| 1 | SAL | 0.5 | 5 |
| 2 | 5 | SAL | 0.5 |
| 3 | 0.5 | SAL | 5 |
| 4 | 0.5 | SAL | 5 |
| 5 | 5 | 0.5 | SAL |
| 6 | 5 | 0.5 | SAL |
| 7 | SAL | 5 | 0.5 |
| 8 | SAL | 5 | 0.5 |
| | | | |
| 9 | SAL | 0.5 | 5 |
| 10 | 5 | SAL | 0.5 |
| 11 | 0.5 | SAL | 5 |
| 12 | 0.5 | SAL | 5 |
| 13 | 5 | 0.5 | SAL |
| 14 | 5 | 0.5 | SAL |
| 15 | SAL | 5 | 0.5 |
| 16 | SAL | 5 | 0.5 |
| | | | |
| 17 | SAL | 0.5 | 5 |
| 18 | 5 | SAL | 0.5 |
| 19 | 0.5 | SAL | 5 |
| 20 | 0.5 | SAL | 5 |
| 21 | 5 | 0.5 | SAL |
| 22 | 5 | 0.5 | SAL |
| 23 | SAL | 5 | 0.5 |
| 24 | SAL | 5 | 0.5 |

### BIBLIOGRAPHY

Abdalla DS, Monteiro HP, Oliveira JA and Bechara EJ., (1986), Clin Chem, 32:805-7
Bowry VW, Mohr D, Cleary J and Stocker R., (1995), J Biol Chem, 270:5756-63
Buckman TD, Kling AS, Eiduson S, Sutphin MS and Steinberg A., (1987), Biol Psychiatry, 22:1349-56
Do KQ, Lauer CJ, Schreiber W, Zollinger M, Gutteck-Amsler U, Cuenod M and Holsboer F., (1995), J Neurochem, 65:2652-62
Do KQ, Trabesinger AH, Kirsten-Kruger M, Lauer CJ, Dydak U, Hell D, Holsboer F, Boesiger P and Cuenod M., (2000), Euro J Neurosci, 12:3721-8
Duffield AJ, Thomson CD, Hill KE and Williams S., (1999), Am J Clin Nutr, 70:896-903
Freedman JH, Ciriolo MR and Peisach J., (1995), J Neurosci, 264:5598-605
Gurney ME, Cutting FB, Zhai P, Doble A, Taylor CP, Andrus PK and Hall ED., (1996), Ann Neurol, 39:147-57
Hagen TM, Ingersoll RT, Lykkesfeldt J, Liu J, Wehr CM, Vinarsky V, Bartholomew JC and Ames AB., (1999), Faseb J, 13:411-8
Louwerse ES, Weverling GJ, Bossuyt PM, Meyjes FE and de Jong JM., (1995), Arch Neurol, 52:559-64
Mahadik SP, Mukherjee S, Scheffer R, Correnti EE and Mahadik JS., (1998), Biol Psychiatry, 43:674-9
Podmore ID, Griffiths HR, Herbert KE, Mistry N, Mistry P and Lunec J., (1998), Nature, 392:559
Yao JK, Reddy RD and van Kammen DP., (1999), Biol Psychiatry, 45:1512-5
Ziegler D and Gries FA., (1997), Diabetes, 46 Suppl 2, S62-6

## Claims

1. Use of a glutathione precursor in the manufacture of a medicament for the treatment of a neuropsychiatric disorder in a mammal, wherein said glutathione precursor induces, upregulates or otherwise augments antioxidant functional activity in said central nervous system.

2. Use according to claim 1 wherein said mammal is human.

3. Use according to claim 2 wherein said neuropsychiatric disorder is schizophrenia, psychosis, bipolar disorder, manic depression, affective disorder, or schizophreniform or schizoaffective disorders, psyschotic depression, drug induced psychosis, delirium, alcohol withdrawal syndrome or dementia induced psychosis.

4. Use according to claim 3 wherein said neuropsychiatric disorder is schizophrenia.

5. Use according to any one of claims 1-4 wherein said glutathione precursor is N-acetyl cysteine.

6. A glutathione precursor for use in the treatment of a neuropsychiatric disorder in a mammal, wherein said glutathione precursor induces, upregulates or otherwise augments antioxidant functional activity in said central nervous system.

7. The precursor of claim 6 wherein said mammal is human.

8. The precursor of claim 7 wherein said neuropsychiatric disorder is schizophrenia, psychosis, bipolar disorder, manic depression, affective disorder, or schizophreniform or schizoaffective disorders, psyschotic depression, drug induced psychosis, delirium, alcohol withdrawal syndrome or dementia induced psychosis.

9. The precursor of claim 8 wherein said neuropsychiatric disorder is schizophrenia.

10. The precursor according to any one of claims 6-9 wherein said glutathione precursor is N-acetyl cysteine.

## Patentansprüche

1. Verwendung eines Glutathionvorläufers zur Herstellung eines Arzneimittels für die Behandlung einer neuropsychiatrischen Störung bei einem Säugetier, wobei der Glutathionvorläufer die funktionelle Antioxidansaktivität im Zentralnervensystem auslöst, aufreguliert oder auf andere Weise erhöht.

2. Verwendung nach Anspruch 1, wobei das Säugetier ein Mensch ist.

3. Verwendung nach Anspruch 2, wobei die neuropsychiatrische Störung eine Schizophrenie, Psychose, bipolare Störung, manische Depression, affektive Störung oder schizophreniforme oder schizoaffektive Störung, psychotische Depression und Drogen-induzierte Psychose, ein Delirium, ein Alkoholentzugssyndrom oder eine Demenzinduzierte Psychose ist.

4. Verwendung nach Anspruch 3, wobei die neuropsychiatrische Störung eine Schizophrenie ist.

5. Verwendung nach einem der Ansprüche 1 bis 4, wobei der Glutathionvorläufer N-Acetylcystein ist.

6. Glutathionvorläufer für die Verwendung bei der Behandlung einer neuropsychiatrischen Störung bei einem Säugetier, wobei der Glutathionvorläufer die funktionelle Antioxidansaktivität im Zentralnervensystem auslöst, aufreguliert oder auf andere Weise erhöht.

7. Vorläufer nach Anspruch 6, wobei das Säugetier ein Mensch ist.

8. Vorläufer nach Anspruch 7, wobei die neuropsychiatrische Störung eine Schizophrenie, Psychose, bipolare Störung, manische Depression, affektive Störung oder schizophreniforme oder schizoaffektive Störung, psychotische Depression und Drogen-induzierte Psychose, ein Delirium, ein Alkoholentzugssyndrom oder eine Demenzinduzierte Psychose ist.

9. Vorläufer nach Anspruch 8, wobei die neuropsychiatrische Störung eine Schizophrenie ist.

10. Vorläufer nach einem der Ansprüche 6 bis 9, wobei der Glutathionvorläufer N-Acetylcystein ist.

## Revendications

1. Utilisation d'un précurseur de glutathione dans la fabrication d'un médicament destiné au traitement d'un trouble neuropsychiatrique chez un mammifère, dans laquelle ledit précurseur de glutathione induit, régule à la hausse ou sinon augmente l'activité fonctionnelle d'antioxydant dans ledit système nerveux central.

2. Utilisation selon la revendication 1, dans laquelle ledit mammifère est un humain.

3. Utilisation selon la revendication 2, dans laquelle ledit trouble neuropsychiatrique est la schizophrénie, la psychose, un trouble bipolaire, la maniaco-dépression, un trouble affectif ou des troubles schizophréniformes ou schizoaffectifs, la dépression psychotique, la psychose induite par médicaments ou drogues, le délire, le syndrome de sevrage d'alcool ou la psychose induite par démence.

4. Utilisation selon la revendication 3, dans laquelle ledit trouble neuropsychiatrique est la schizophrénie.

5. Utilisation selon l'une quelconque des revendications 1 à 4, dans laquelle ledit précurseur de glutathione est la N-acétyl cystéine.

6. Précurseur de glutathione à utiliser dans le traitement d'un trouble neuropsychiatrique chez un mammifère, dans lequel ledit précurseur de glutathione induit, régule à la hausse ou sinon augmente l'activité fonctionnelle d'antioxydant dans ledit système nerveux central.

7. Précurseur selon la revendication 6, dans lequel ledit mammifère est un humain.

8. Précurseur selon la revendication 7, dans lequel ledit trouble neuropsychiatrique est la schizophrénie, la psychose, un trouble bipolaire, la maniaco-dépression, un trouble affectif ou des troubles schizophréniformes ou schizoaffectifs, la dépression psychotique, la psychose induite par médicaments ou drogues, le délire, le syndrome de sevrage d'alcool ou la psychose induite par démence.

9. Précurseur selon la revendication 8, dans lequel ledit trouble neuropsychiatrique est la schizophrénie.

10. Précurseur selon l'une quelconque des revendications 6 à 9, dans lequel ledit précurseur de glutathione est la N-acétyl cystéine.
